# FASCICULE DE BREVET EUROPEEN

(11) **EP 1 246 889 B1**
(45) Date de publication et mention de la délivrance du brevet: **21.04.2010**
(21) Numéro de dépôt: 00993626.1
(22) Date de dépôt: 28.12.2000
(51) Int. Cl.: C10G 27/12, C10G 67/12, C10G 53/14

(54) **PROCEDE DE DESULFURATION DES DERIVES DU THIOPHENE CONTENUS DANS DES CARBURANTS**
VERFAHREN ZUR ENTSCHWEFELUNG VON IN KRAFTSTOFFEN ENTHALTENEN THIOPHENDERIVATEN
METHOD FOR DESULPHURIZING THIOPENE DERIVATIVES CONTAINED IN FUELS

(30) Priorité: 28.12.1999 FR 9916559
(43) Date de publication de la demande: 09.10.2002
(73) Titulaire: TOTAL RAFFINAGE MARKETING, 92800 Puteaux (FR)
(72) Inventeur: RABION, Alain, F-64000 Pau (FR); FAJULA, François, F-34820 Teyran (FR); BERNARD, Jean-René, F-69360 Serezin du Rhône (FR); HULEA, Vasile, Faculté de Chimie Industrielle, 6600 Iasi (RO)
(74) Mandataire: Roger, Walter
(86) Numéro de dépôt international: PCT/FR2000/003711
(87) Numéro de publication internationale: WO 2001/048119

(56) Documents cités:
- EP-A- 0 029 472
- EP-A- 0 115 382
- EP-A- 0 565 324
- FR-A- 1 595 726
- US-A- 3 647 683
- US-A- 3 816 301
- DATABASE WPI Section Ch, Week 198632 Derwent Publications Ltd., London, GB; Class H04, AN 1986-211195 XP002147552 & SU 1 203 102 A (AS SIBE CHEM PETROL), 7 janvier 1985 (1985-01-07)
- DATABASE WPI Section Ch, Week 199042 Derwent Publications Ltd., London, GB; Class H04, AN 1990-318686 XP002147553 & SU 1 549 985 A (PETROCHEM PROD RES), 15 mars 1990 (1990-03-15)

## Description

La présente invention concerne un procédé de désulfuration de carburants, c'est-à-dire à la fois de gazoles, de kérosènes et d'essences. Il vise en particulier la désulfuration de carburants contenant des composés du thiophène.

La présence de soufre dans les carburants constitue un problème considéré aujourd'hui comme majeur pour l'environnement. En effet, par combustion, le soufre est converti en divers oxydes de soufre, qui peuvent se transformer en acides, contribuant ainsi à la formation de pluies acides.

Généralement, les raffineries utilisent des procédés d'hydrodésulfuration catalytique pour abaisser la teneur en soufre des carburants. Ainsi, les gazoles issus directement de la distillation sont traités dans des réacteurs travaillant entre 300 et 400 °C, sous une pression d'hydrogène comprise entre 30 et 100.10⁵ Pa (entre 30 et 100 bars) et à des vitesses spatiales horaires comprises entre 0,5 et 4 h⁻¹. Les composés soufrés du carburant réagissent avec l'hydrogène au contact du catalyseur disposé en lit fixe et constitué de sulfures de métaux des groupes VI et VIII déposés sur de l'alumine, par exemple des sulfures de cobalt et de molybdène ou des sulfures de nickel et de molybdène. Du fait des conditions opératoires et de la consommation d'hydrogène, ces procédés peuvent être coûteux en investissement et en fonctionnement, et d'autant plus dans le cas où l'on cherche à produire des carburants à très basse teneur en soufre. Ainsi, pour désulfurer un carburant contenant initialement 1 % en poids de soufre jusqu'à une teneur en soufre comprise entre 0,05 et 0,005 % en poids, la taille du réacteur peut être multipliée par 4 et il faut également augmenter la quantité d'hydrogène nécessaire à la réaction. On comprend ainsi pourquoi il est particulièrement difficile d'éliminer des traces de soufre, en particulier si le soufre est contenu dans des molécules réfractaires comme le dibenzothiophène alkylé en position 4, ou 4 et 6.

Dans certains pays, comme la Suède, les Etats Unis, (notamment en Californie) et d'autres, la teneur en soufre total des gazoles est déjà limitée à 0,005 % en poids. Cette limitation pourrait se généraliser à terme dans les pays de l'OCDE.

Parallèlement, la teneur en soufre total dans les essences en France est limitée à 0,05 % en poids, mais cette limite devrait bientôt être abaissée à 0,005 % en poids ou moins en 2005, et cela dans toute l'Europe.

Les essences, contrairement aux gazoles, ne sont pas obtenues seulement par distillation directe, mais également par divers procédés tels que le réformage des naphtas, l'isomérisation des naphtas légers, l'alkylation des butane ou propane produisant l'isooctane, la méthoxylation de l'isobutène et le craquage catalytique des distillats sous vide ou des résidus atmosphériques. Le craquage fournit entre 20 et 60 % en poids de l'essence finale et ce sont les essences produites via ce procédé qui amènent les composés soufrés dans l'essence, si on excepte les faibles quantités de soufre présentés dans les essences de distillation directe.

Pour désulfurer ces essences de craquage, il est classique de mettre en oeuvre des procédés semblables à ceux décrits pour l'hydrodésulfuration des gazoles, pour lesquels les conditions opératoires sont plus sévères en pression d'hydrogène, en vitesse spatiale et en température. Là encore, ces procédés sont coûteux en investissement, en fonctionnement et en hydrogène, compte tenu des teneurs en soufre que l'on veut atteindre. Il est cependant possible d'atteindre, de façon classique, des teneurs en soufre total dans ces essences de craquage comprises entre 0,005 et 0,03 % en poids, selon la quantité d'hydrocarbures traités et la sévérité de l'hydrotraitement de ces hydrocarbures avant l'étape de craquage. En l'absence d'hydrotraitement, la teneur en soufre total dans l'essence de craquage pourrait atteindre jusqu'à 0,1% en poids. Pour réduire cette teneur en soufre, il est possible d'ajouter au catalyseur de craquage des additifs décomposant les mercaptans et les sulfures formés dans l'essence recueillie. Malheureusement, ces additifs n'ont aucun effet ou qu'un effet limité sur les dérivés benzothiophèniques, même si les hydrocarbures chargés dans l'unité de craquage catalytique ont été préalablement hydrodésulfurés, c'est-à-dire que les mercaptans et les sulfures en ont été éliminés.

Un inconvénient majeur de l'hydrodésulfuration des essences de craquage est que, parallèlement à la désulfuration, il y a hydrogénation partielle des oléfines. Or, ces oléfines sont responsables du bon indice d'octane de l'essence, et leur disparition se traduit par une baisse de cet indice, donc une qualité moindre de l'essence. Pour compenser cette perte, on peut soit introduire d'autres constituants pour améliorer cet indice, soit retraiter l'essence elle-même pour augmenter cet indice. Comme l'ajout d'un nouveau traitement ou de nouveaux composés dans l'essence en vue d'en améliorer la qualité grève d'autant son prix de revient, on comprend l'avantage que peut présenter un procédé de traitement visant à désulfurer les composés soufrés et, plus sélectivement, les composés benzothiophèniques, laissant les molécules non soufrées intactes et limitant l'utilisation d'hydrogène.

Les procédés d'oxydation sélective des composés soufrés peuvent remplir ce rôle. Parmi les méthodes et procédés développés pour réduire la quantité de soufre présent dans les carburants sous forme de dérivés du thiophène, l'oxydation par des peroxydes organiques, des hydroperoxydes organiques, le peroxyde d'hydrogène et des peracides organiques en présence ou en l'absence de catalyseurs à base de composés organométalliques ou d'oxydes métalliques, a été envisagée (voir US 3 668 117, US 3 565 793, EP 0 565 324 et les publications de T.A. KOCH, K.R. KRAUSE, L. EMANZER, H. MEHDIZADEH, J.M. ODOM, S.K. SENGUPTA, New J. Chem., 1996, 20, 163-173 et de F.M. COLLINS, A.R. LUCY, C. SHARP, J. of Molecular Catalysis A : Chemical 117 (1997) 397-403).

Pour les procédés mettant en oeuvre des catalyseurs métalliques à base de molybdène et de tungstène en présence de peroxyde d'hydrogène, on opère à des températures supérieures à 60 °C et il y a surconsommation de peroxyde d'hydrogène, une partie de cet oxydant étant décomposée par le catalyseur utilisé. L'utilisation des peracides, oxydants très puissants, obtenus par réaction de peroxyde d'hydrogène et d'un acide carboxylique tel que l'acide formique ou l'acide acétique, est très dangereuse à ces températures en milieux hydrocarbonés, en raison des risques d'explosion en cas de choc ou en présence de lumière. De plus, ils sont moins efficaces que le peroxyde d'hydrogène et moins sélectifs vis-à-vis des composés soufrés, de sorte qu'ils peuvent oxyder notamment les oléfines.

Dans toutes ces méthodes et procédés, on transforme les dérivés du thiophène en leur forme sulfonée et/ou sulfonique. Cependant, pour certains de ces composés même si on travaille à forte température, la réaction est relativement lente et la conversion totale n'est pas atteinte en moins d'une heure, sinon en utilisant de très fortes concentrations d'oxydants, souvent très supérieures aux quantités nécessaires à l'oxydation quasi-stoechiomètrique des dérivés soufrés.

D'autres procédés d'oxydation utilisent des phtalocyanines ou des polyphtalocyanines métallées en présence d'oxygène ou d'ozone pour transformer les mercaptans et l'H₂S contenus dans les produits pétroliers en disulfures organiques, comme décrit, par exemple, dans les brevets N° US 3 565 959 et US 3 039 855. Cependant de tels procédés ne permettent pas d'oxyder les composés du thiophène qui demeurent ainsi dans les produits pétroliers. En outre, appliqués aux essences de craquage catalytique, ces procédés favorisent la formation de gommes par polymérisation des oléfines, ce qui rend ces essences impropres.

La présente invention vise donc à proposer un procédé de désulfuration des carburants contenant des composés thiophéniques, sans diminution de l'indice du nombre d'octane ou du nombre de cétane, parfois même avec augmentation de ces indices. Elle concerne particulièrement le traitement de finition des gazoles hydrotraités, des kérosène et des essences de craquage catalytique, fortement concentrés en composés thiophèniques réfractaires aux hydrogénations.

La présente invention a donc pour objet un procédé tel que défini dans le libellé de la revendication independente de désulfuration sélective des composés thiophéniques contenus dans les hydrocarbures issus de la distillation du pétrole brut, raffinés ou non, consistant à oxyder les atomes de soufre thiophénique en sulfone en présence d'un agent oxydant et à séparer les composés sulfonés des dits hydrocarbures, caractérisé en ce qu'on oxyde les composés thiophéniques en un milieu turbulent biphasique comprenant une phase hydrocarbonée et une phase aqueuse, en présence d'au moins un agent oxydant soluble dans au moins une des deux phases et d'au moins un catalyseur métallique choisi parmi les phtalocyanines métalliques, éventuellement substituées par des groupements alkyle comprenant de 1 à 4 atomes de carbone et/ou des groupements sulfoniques, les catalyseurs comprenant un support du groupe constitué par les silices, les alumines, les zircones, les aluminosilicates amorphes ou cristallins, les aluminophosphates et les solides mésoporeux, seuls ou en mélange, comprenant éventuellement au moins un métal du groupe constitué par le titane, le zirconium, le vanadium, le chrome, le molybdène, le tungstène, le fer, le manganèse et le tungstène, ces métaux pouvant être introduits dans le réseau du support ou imprégnés sous forme complexée ou non. Parmi ces catalyseurs, les catalyseurs au titane non oxydé sont préférés.

D'autres caractéristiques de l'objet de la demande sont décrites dans les revendications dependentes.

Le procédé selon l'invention présente l'avantage de permettre une oxydation sélective du soufre thiophènique en sulfone. La séparation des sulfones des hydrocarbures est immédiate, celles-ci se retrouvant dans la phase aqueuse. En outre, cette oxydation n'a aucun effet sur les oléfines, ce qui ne modifie pas, dans les essences de craquage catalytique, l'indice d'octane ni la teneur en composés aromatiques non soufrés. Le procédé d'oxydation selon l'invention améliore en outre le nombre de cétane des gazoles.

Ces effets spécifiques sont liés à l'effet de synergie des agents oxydants retenus et des catalyseurs utilisés.

Ainsi, dans le cadre de la présente invention, l'agent oxydant est choisi dans le groupe constitué par les peroxydes organiques, le peroxyde d'hydrogène, les hydroperoxydes organiques, les peracides et les persulfates alcalins et alcalino-terreux.

Pour compléter l'extraction, c'est-à-dire le passage des composés thiophéniques sulfonés de la phase hydrocarbonée dans la phase aqueuse et accélérer la réaction d'oxydation, on ajoute au milieu réactionnel biphasique un solvant miscible dans l'eau et peu miscible dans les hydrocarbures, choisi dans le groupe constitué par les alcanols comprenant de 1 à 4 atomes de carbone, l'acétonitrile, le diméthyl formamide, le nitrométhane, le nitrobenzène, dans un rapport eau/solvant compris entre 1/99 à 99/1 et de préférence entre 25/75 et 75/25. Pour récupérer les composés sulfonés issus de la réaction d'oxydation, on pourrait également procéder par distillation et/ou absorption sur un oxyde réfractaire de type alumine ou silice, et/ou par précipitation de ces composés oxydés, selon des procédures connues, telles que décrites dans les brevets EP 0 585 324 et EP 0 482 841.

Dans un mode préféré de mise en oeuvre du procédé de l'invention, le rapport molaire catalyseur métallique (exprimé en métal) /oxydant varie de 1/10⁵ à 100/ 1 dans le milieu réactionnel biphasique, la température de réaction est comprise entre la température ambiante et 90°C et de préférence entre 50 et 90°C, sous pression atmosphérique, et le pH de la solution aqueuse est maintenu inférieur à 12 et de préférence compris entre 4 et 9.

Dans un mode particulier de mise en oeuvre de l'invention, parmi les catalyseurs métalliques constitués de phtalocyanines métalliques, on préfère les phtalocyanines de fer, éventuellement substituées par des groupements alkyles comprenant de 1 à 4 atomes de carbone et/ ou des groupements sulfones, en solution aqueuse ou supportées sur une phase solide choisie dans le groupe des oxydes métalliques réfractaires tels que l'alumine, la zircone, la silice, le tungstate, les argiles et les résines organiques telles que les résines cationiques fonctionnalisées par des groupements ammonium. Dans ce mode de réalisation, les phtalocyanines métalliques ne sont pas sous forme substituée par des groupements sulfoniques et peuvent être liées au support par des liaisons ioniques ou covalentes de type sulfonamide.

Lorsque le catalyseur métallique est un catalyseur au titane non oxydé, on préfère les zéolithes au titane, sans titane extra-réseau, de diamètre de pores d'au moins 0,65 nm, et les composites mésoporeux au titane, et encore plus particulièrement la zéolithe bêta au titane. Ces zéolithes peuvent être préparées en mettant en oeuvre le procédé décrit dans le brevet EP 0 842 114.

Dans ce mode particulier de mise en oeuvre de l'invention, le rapport molaire catalyseur métallique(exprimé en métal) /oxydant varie de 1/10 à 1 /40 dans le milieu réactionnel biphasique.

Selon l'invention, les oxydants sont choisis parmi les composés de formule générale R₁OOR₂, dans laquelle R₁ et R₂ sont identiques ou différents, choisis parmi l'hydrogène et les groupements alkyle, linéaires ou ramifiés, comprenant de 1 à 30 atomes de carbone.

Dans un mode préféré, l'oxydant de formule R₁OOR₂ est choisi dans le groupe constitué par le peroxyde d'hydrogène, le terbutyl hydroperoxyde et le tertbutylperoxyde. Les oxydants préférés sont le terbutylperoxyde et le peroxyde d'hydrogène, ce dernier étant largement préféré pour son effet peu polluant.

D'autres oxydants de l'invention, les peracides de formule R₃COOOH, sont choisis tels que R₃ est l'hydrogène ou un groupement alkyl linéaire ou ramifié comprenant de 1 à 30 atomes de carbone. Ils sont choisis de préférence dans le groupe constitué par l'acide peracétique, l'acide performique et l'acide perbenzoïque. Pour éviter tout problème d'explosion, ils sont formés in situ par addition progressive d'une petite quantité d'un mélange peroxyde d'hydrogène/acide carboxylique.

Quels que soient l'oxydant et le catalyseur utilisés, le rapport massique solution aqueuse/hydrocarbures varie de 10/ 1 à 1/10 De préférence, on opérera avec un rapport variant de 2/1 à 1/5.

Un deuxième objet de l'invention est l'application du procédé défini ci-dessus au traitement spécifique de finition des essences issues du craquage catalytique ou encore au traitement des gazoles ayant été préalablement hydrotraités et des kérosènes, pour une meilleure économie du procédé.

Les exemples ci-après visent à illustrer l'efficacité du procédé de l'invention, sans en limiter la portée.

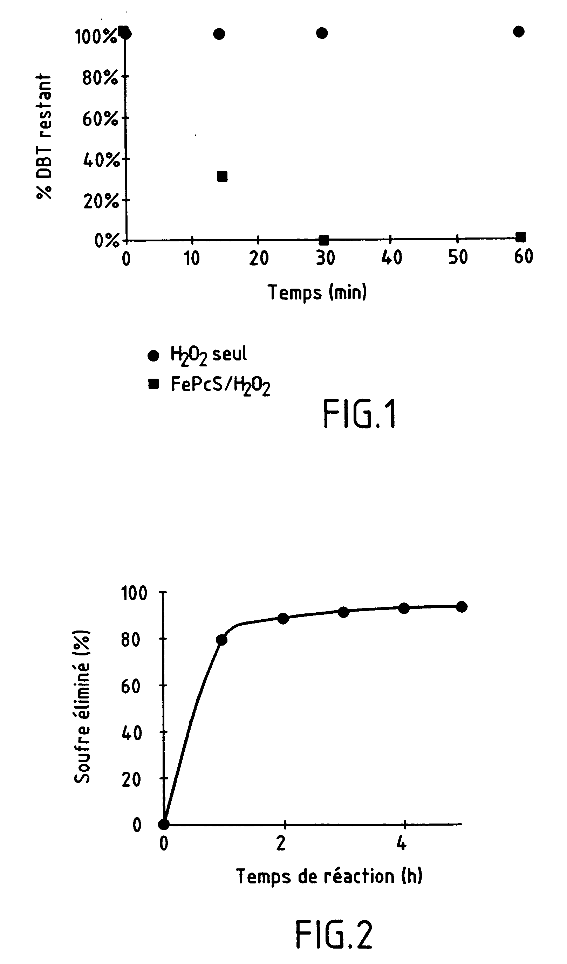

Dans ces exemples, on se référera aux figures 1 et 2 des dessins annexés, qui seront explicitées respectivement dans les Exemples 1 et V correspondants.

### EXEMPLE I

Le présent exemple vise à montrer l'activité de la combinaison phtalocyanine métallée/peroxyde d'hydrogène vis-à-vis de l'oxydation des dérivés du thiophène présents dans les carburants, principalement le benzothiophène (BT), le dibenzothiophène (DBT) et le 4,6 diméthyldibenzothiophène (DMBT).

Les phtalocyanines métallées utilisées sont des sulfophtalocyanines de fer (FePcS), de cobalt (CoPcS) et de Nickel (NiPcS). Les essais ont lieu en milieu bi-phasique organo-(acétonitrile/eau) aqueux à pH 7,7, le rapport oxydant phtalocyanine métallée étant 20/1, la température étant la température ambiante de 20°C et la pression étant la pression atmosphérique, le mélange étant agité.

A titre d'essais comparatifs et pour montrer l'efficacité du catalyseur, on effectue des oxydations de ces mêmes dérivés thiophéniques, mais, d'une part, en présence d'un catalyseur constitué d'une zéolithe TS-1 (zéolithes dont le diamètre des pores est inférieur à 0,6 nm) et de peroxyde d'hydrogène H₂O₂ et, d'autre part, en présence de phtalocyanine de fer et de l'oxygène de l'air.

Les dérivés du thiophène à oxyder sont introduits dans l'acétonitrile à 1 mmole/l. Le Tableau 1 ci-après rassemble les résultats en pourcentage d'oxydation obtenu au bout de 30 minutes de réaction.

**TABLEAU 1**

| Catalyseur/ Oxydant | Concentration oxydant (eqS) | % Oxydation en sulfone | | |
|---|---|---|---|---|
| | | BT (1mM) | DBT(1mM) | DMBT(1mM) |
| H₂O₂ | 2 | 3 | <1 | <1 |
| FePcS (air) | | 0 | 0 | 0 |
| FePcS/H₂O₂ | 2 | 90 | 95 | 95 |
| FePcS/H₂O₂ | 3 | 100 | 100 | 100 |
| CoPcS/H₂O₂ | 2 | <1 | <1 | <1 |
| NiPcS/H₂O₂ | 2 | <1 | <1 | < 1 |
| TS-1/H₂O₂ | 2 | 0 | 0 | 0 |

La figure 1 est une courbe illustrant l'évolution de la teneur en DBT (dibenzothiophène) restant dans le milieu biphasique acétonitrile/eau en fonction du temps.

A température ambiante et à pression atmosphérique, l'oxydation des dérivés du thiophène par les peroxydes seuls ou par la phtalocyanine métallique sulfonée seule en milieu aéré, est inefficace.

Par contre les couples phtalocyanines de fer/peroxydes catalysent très efficacement l'oxydation des dérivés BT, DBT et DMBT du thiophène en sulfone. Il est à noter que le DMBT, substrat difficile à oxyder en raison de l'encombrement stérique des groupes méthyle en position β du soufre est oxydé aussi rapidement que le DBT.

Par contre, les sulfophtalocyanines de cobalt et de nickel, ainsi que la TS-1 sont inefficaces pour une telle oxydation, dans les conditions de réaction.

### EXEMPLE II

Le présent exemple vise à présenter l'oxydation du dibenzothiophène en catalyse biphasique liquide/liquide, en choisissant l'heptane comme solvant de la phase organique. La phase aqueuse est composée de tampon de pH 7,7 constitué de phosphate et d'acétone, de manière à solubiliser la sulfophtalocyanine de fer et un minimum de DBT (partition initiale du DBT 95:5, phase organique / phase aqueuse).

On opère dans les conditions suivantes :
DBT : 1 mmol/l; FePcS 0,1 mmol/l ; H₂O₂ : ajouté en continu 5 eq/h ; température : 20°C ; pression atmosphérique ; agitation magnétique.

Dans les conditions de réaction utilisées, l'oxydation catalysée du DBT est totale après 30 min de réaction, soit après l'ajout de 2,5 équivalents de H₂O₂. Le DBT est converti entièrement en sulfone correspondant lequel est stable vis à vis du système oxydant utilisé et est partagé entre les deux phases (portion finale de la sulfone 1/3, phase organique / phase aqueuse).

### EXEMPLE III

Le présent exemple concerne l'oxydation en mélange triphasique, lorsque le catalyseur est un catalyseur au titane non oxydé sous forme solide dispersé dans le mélange réactionnel biphasique . Deux types de catalyseur ont été testés : Ti-bêta (zéolithe avec une structure BEA) et Ti-HMS (composite mésoporeux au titane). La Ti-bêta (sans titane extra-réseau) a été obtenue à partir d'une zéolithe du commerce post-traitée selon la procédure décrite dans le brevet EP 0 842 114.

Le composite mésoporeux au titane a été obtenu par co-précipitation, en milieu fortement acide, de silicate et d'oxyde de titane, en présence de tensioactif non ionique de type pluronic

Les principales caractéristiques des catalyseurs sont présentées dans le Tableau 2 ci-après.

**TABLEAU 2**

| Catalyseur | Ti/(Ti+Si) mole/mole | Surface BET m²/g | Diamètre des particules, µ | Vmp (ml/g) |
|---|---|---|---|---|
| Ti-bêta | 0,008 | 470 | 0,3 | 0,28 |
| Ti-HMS | 0,024 | 838 | 10 | 0,42 |

L'oxydant utilisé est le peroxyde d'hydrogène. La fraction à oxyder est une solution de benzothiophène et dibenzothiophène dans du n-décane. On introduit dans un réacteur de 60 ml le milieu de réaction contenant 0,5 mmole de benzothiophène, 0,5 mmole de dibenzothiophène, 20 ml de n-décane, 1 ml de solution aqueuse contenant 30 % en poids de peroxyde d'hydrogène, 100 mg de catalyseur et 20 ml d'un solvant non miscible dans le n-décane, qui peut être un alcool, l'acétonitrile ou l'eau. Le milieu est agité énergiquement au moyen d'un agitateur magnétique et l'ensemble est maintenu à une température de 70°C (ou 64,5°C si le méthanol est le solvant), sous pression atmosphérique.

Après réaction, les phases hydrocarbonées et aqueuses sont séparées par simple décantation.

Les conversions des composés soufrés sont présentées dans le Tableau 3 ci-après, après cinq heures de réaction.

**TABLEAU 3**

| Catalyseur | Solvant | Conversion, % | |
|---|---|---|---|
| | | Benzothiophène | Dibenzothiophène |
| Ti-HMS | acétonitrile | 56,0 | 67,0 |
| Ti-bêta | méthanol | 100 | 93,7 |
| Ti-bêta | acétonitrile | 96,7 | 91,0 |
| Ti-bêta | eau | 64,3 | 25,0 |

On constate d'après ces résultats que les deux catalyseurs sont efficaces pour oxyder les thiophènes. L'efficacité du solvant est liée à la solubilité des produits d'oxydation. Ainsi, ils sont beaucoup plus solubles dans le méthanol et l'acétonitrile que dans l'eau.

### EXEMPLE IV

Le présent exemple vise l'oxydation en mélange triphasique, lorsque le catalyseur n'est pas un catalyseur au titane. On opère comme décrit à l'Exemple III.

Parmi les catalyseurs mis en oeuvre dans cet exemple, les catalyseurs à base de solides mésoporeux HMS sont obtenus par co-précipitation en milieu fortement acide, de silicate et d'oxyde de vanadium, de tungstène ou de molybdène, en présence de tensioactif non ionique de type pluronic.

Les catalyseurs à base d'alumine ou de zircone sont obtenus par imprégnation par voie humide de métatungstate d'ammonium ou de vanadate en solution aqueuse, puis séchage et enfin calcination à 500°C.

Le Tableau 4 ci-après rassemble les résultats obtenus avec ces catalyseurs.

**TABLEAU 4**

| Catalyseur | Solvant | Conversion, % | |
|---|---|---|---|
| | | Benzothiophène | Dibenzothiophène |
| V-HMS | méthanol | 40,0 | 45.0 |
| V-HMS | acétonitrile | 51 | 55 |
| Mo-HMS | acétonitrile | 32 | 36 |
| W-HMS | acétonitrile | 20 | 27 |

Il est clair, d'après ce tableau et l'exemple précédent, que l'efficacité de la désulfuration des dérivés di benzothiophènique est très dépendant du compromis, nature du support, nature du métal et nature du solvant d'extraction des sulfones formées. Dans les conditions opératoires de l'exemple III, les catalyseurs au titane semblent être les plus efficaces.

### EXEMPLE V

Le présent exemple vise à décrire la cinétique d'oxydation de composés soufrés contenus dans le kérosène. Le catalyseur utilisé est la Ti-bêta, dont les caractéristiques sont présentées dans l'Exemple III. La fraction traitée est un kérosène contenant 1310 ppm de soufre, présent dans la plupart des composés soufrés sous forme de composés thiophéniques.

On introduit dans un réacteur de 100 ml le milieu de réaction contenant 40 ml de kérosène, 0,35 ml de solution aqueuse à 30% en poids de peroxyde d'hydrogène, 1 g de catalyseur et 20 ml d'acétonitrile. Le milieu est agité énergiquement au moyen d'un agitateur magnétique et l'ensemble est maintenu à une température de 60°C, sous pression atmosphérique. Le kérosène est ensuite lavé par de l'acétonitrile pour terminer la séparation avant de mesurer la teneur en soufre total.

Le taux de désulfuration du kérosène en fonction du temps de réaction est présenté sur la figure 2.

On constate d'après cette figure qu'au bout de 2 heures, 90% du soufre est éliminé. Les composés soufrés oxydés passent intégralement dans la phase contenant l'acétonitrile et l'eau.

### EXEMPLE VI

Le présent exemple vise l'oxydation en mélange triphasique lorsque le catalyseur est un catalyseur au titane non oxydé sous forme solide dispersé dans le mélange réactionnel biphasique. Deux types de catalyseur ont été testés : Ti-bêta et Ti-HMS, dont les caractéristiques sont présentées dans l'Exemple III.

La fraction traitée est un kérosène contenant 1310 ppm de soufre, pour l'essentiel sous forme de composés thiophéniques.

On introduit dans un réacteur de 100 ml, le milieu de réaction contenant 40 ml d'hydrocarbures, 2 ml de solution aqueuse (30% gr) de peroxyde d'hydrogène, 200 mg de catalyseur et 20 ml d'un solvant non miscible avec les hydrocarbures, qui peut être un alcool, l'acétonitrile ou l'eau. Le milieu est agité énergiquement au moyen d'un agitateur magnétique et l'ensemble est maintenu à une température de 70°C, sous pression atmosphérique.

Après la réaction, les trois phases (kérosène, solvant + eau, catalyseur) ont été séparées par filtration et décantation. Aucune autre opération n'a été faite sur les fractions avant de les analyser.

L'efficacité des catalyseurs pris en en présence de peroxyde d'hydrogène est mesurée par la diminution du soufre dans la phase hydrocarbonée surnageante. Les résultats sont donnés dans le tableau 5 ci-après.

**TABLEAU 5**

| Catalyseur | Solvant | Temps de réaction (h) | Phase analysée | Soufre, ppm | %Soufre éliminé |
|---|---|---|---|---|---|
| - | Acétonitrile | Extraction | kérosène | 1220 | 7,0 |
| Ti-HMS | Acétonitrile | 9 | kérosène | 190 | 85.5 |
| | | 9 | MeCN | 2500 | |
| Ti-bêta | Acétonitrile | 5 | kérosène | 80 | 94,0 |
| | | 5 | MeCN | 2300 | |
| Ti-bêta | Ethanol | 5 | kérosène | 390 | 70,2 |
| | | 10 | | 300 | 77,0 |
| | | 24 | | 250 | 81,0 |
| | | 24 | kérosène lavé à l'acétonitrile | 80 | 94,0 |
| | | 24 | EtOH | 1800 | |
| Ti-bêta | H₂O | 10 | kérosène | 840 | 36,0 |
| | | 10 | kérosène lavé à l'acétonitrile | 300 | 77,1 |
| | | 10 | H₂O | 450 | |

On constate, d'après ce tableau, que les deux catalyseurs sont aussi efficaces pour oxyder le soufre présent dans le kérosène. Les produits d'oxydation (sulfones) sont très peu solubles dans les hydrocarbures et ils passent dans le solvant. L'efficacité du solvant est liée à la solubilité des sulfones : acétonitrile > éthanol > eau.

## Revendications

1. Procédé de désulfuration sélective des composés thiophéniques contenus dans lies hydrocarbures issus de la distillation du pétrole brut, raffinés ou non, consistant à oxyder les atomes de soufre thiophénique en sulfone en présence d'un agent oxydant et à séparer les composés sulfonés des dits hydrocarbures, **caractérisé en ce qu'**on oxyde les composés thiophéniques dans un milieu turbulent biphasique comprenant une phase hydrocarbonée et une phase aqueuse, en présence d'au moins un agent oxydant soluble dans au moins une des deux phases, choisi dans le groupe constitué par les peroxydes organiques, le peroxyde d'hydrogène, les hydroperoxyde organiques, les peracides et les persulfates alcalins et alcalino-terreux, et d'au moins un catalyseur métallique choisi parmi les phtalocyanines métalliques, éventuellement substituées par des groupements alkyle comprenant de 1 à 4 atomes de carbone et/ou des groupements sulfoniques, et les catalyseurs comprenant un support du groupe constitué par les silices, les alumines, les zircones, les alummosilicates amorphes ou cristallins, les alumihophosphates et les solides mésoporeux, seuls ou en mélange, comprenant éventuellement au moins un métal du groupe constitué par le titane, le zirconium, le vanadium, le chrome, le molybdène, le tungstène, le fer, le manganèse et le tungstène, ces métaux pouvant être introduits dans le réseau du support ou imprégnés sous forme complexée ou non, les catalyseurs au titane non oxydé étant préférés, la séparation se produisant simultanément à l'oxydation, par extraction, c'est-à-dire par le passage des composés thiophéniques sulfonés de la phase hydrocarbonée dans la phase aqueuse.

2. Procédé selon la revendication 1, **caractérisé en ce que** l'on ajoute au milieu réactionnel biphasique un solvant d'extraction choisi dans le groupe constitué par les alcanols comprenant de 1 à 4 atomes de carbones, l'acétonitrile, le diméthyl formamide, le nitrométhane, le nitrobenzène, dans un rapport eau /solvant compris de 1/99 à 99/1 et de préférence entre 25/75 et 75/25.

3. Procédé selon l'une des revendications 1 et 2, **caractérisé en ce que** le rapport molaire catalyseur métallique (exprimé en métal)/oxydant est compris entre 1/10⁵ et 100/1, que la réaction démarre à la température ambiante sous pression atmosphérique et que le pH de la solution aqueuse est maintenu inférieur à 12 et de préférence compris entre 4 et 9.

4. Procédé selon l'une des revendications 1 à 3, **caractérisé en ce que** la phtalocyanine métallique est une phtalocyanine de fer, éventuellement substituée par des groupements alkyle comprenant de 1 à 4 atomes de carbone et/ou des groupements sulfoniques en solution aqueuse.

5. Procédé selon l'une des revendications 1 à 3, **caractérisé en ce que** la phtalocyanine métallique est une phtalocyanine de fer, éventuellement substituée par des groupements alkyle comprenant de 1 à 4 atomes de carbone et/ou des groupements sulfoniques, supportée sur une phase solide choisie dans le groupe des oxydes métalliques réfractaires tels que l'alumine, la zircone, la silice, le tungstate , les argiles et les résines organiques telles que les résines cationiques fonctionnalisées par des groupements ammonium.

6. Procédé selon l'une des revendications 1 à 3, **caractérisé en ce que** le catalyseur au titane est choisi dans le groupe constitué par les zéolithes au titane sans titane extra-réseau, de diamètre de pores supérieur ou égal à 0,65 nm, de préférence la zéolithe bêta au titane, et les composites méso-poreux au titane.

7. Procédé selon la revendication 6, **caractérisé en ce que** le rapport molaire catalyseur métallique (exprimé en métal)/oxydant varie de 1/10 à 1/40.

8. Procédé selon l'une des revendications 1 à 7, **caractérisé en ce que** l'oxydant est un composé de formule générale R₁OOR₂, dans laquelle R₁ et R₂ sont choisis identiques ou différents dans le groupe constitué par l'atome d'hydrogène, les groupements alkyle, linéaires ou ramifiés comprenant de 1 à 30 atomes de carbone.

9. Procédé selon la revendication 8, **caractérisé en ce que** l'oxydant est choisi dans le groupe constitué par le peroxyde d'hydrogène, le terbutyl hydroperoxyde et le terbutyl peroxyde.

10. Procédé selon l'une des revendications 8 et 9, **caractérisé en ce que** l'oxydant est le peroxyde d'hydrogène.

11. Procédé selon l'une des revendications 1 à 7, **caractérisé en ce que** l'oxydant est un peracide de formule R₃COOOH, dans laquelle R₃ est l'hydrogène ou un groupement alkyle linéaire ou ramifié comprenant de 1 à 30 atomes de carbone.

12. Procédé selon la revendication 11, **caractérisé en ce que** l'oxydant est choisi dans le groupe constitué par l'acide peracétique, l'acide performique et l'acide perbenzoïque.

13. Procédé selon l'un des revendications 1 à 12, **caractérisé en ce que** le rapport massique solution aqueuse/hydrocarbures est compris entre 10/ 1 et 1/10, et de préférence entre 2/1 et 1/5.

14. Application du procédé selon l'une des revendications 1 à 13, à la désulfuration des gazoles hydrotraités, des kérosènes et des essences, notamment des essences issues du craquage catalytique.

## Claims

1. Method of selective desulfurizing of thiophene compounds contained in the hydrocarbons derived the distillation of crude oil, refined or not, consisting in oxidizing the thiophene sulfur atoms into sulfone in the presence of an oxidizing agent and in separating the sulfone compounds of said hydrocarbons, **characterized in that** we oxidize the thiophene compounds in a two-phase turbulent phase comprising a hydrocarbon phase and an aqueous phase, in the presence of at least one oxidizing agent soluble in at least one of two phases, chosen the group consisting of organic peroxides, hydrogen peroxide, organic hydroperoxides, peracids and alkalin an alkyl-earthy persulfates and at least one metal catalyst chosen from the metal phtalocyanines, possibly substituted by alkyl groups comprised of 1 to 4 carbon atoms and/or sulfonic groups, and the catalysts comprised of a support of the group consisting of silicas, aluminas, zirconias, amorphous or crystalline aluminosilicates, aluminophosphates and mesoporous solids, alone or mixed, possibly comprised of at least one metal of the group consisting of titanium, zirconium, vanadium, chrome, molybdenum, tungstene, iron, manganese and tungstene, where said metals can be introduced into the support network or impregnated in complex or non complex form, the non oxidized titanium catalysts are preferred, the separation taking place simultaneously to the oxidation by extraction, e.g, by the passing of the sulfonated thiophene compounds from the hydrocarbon phase to the aqueous phase.

2. Method according to claim 1, **characterized in that** we add an extraction solvent chosen from the group consisting of alcanols comprised of 1 to 4 carbon atoms, acetonitrile, formamide dimethyl, nitromethane, nitrobenzene, to the two-phase reaction medium, in a water/solvent ratio ranging between 1/99 and 99/1 and preferably between 25/75 and 75/25.

3. Method according to claim 1 or 2, **characterized in that** the metal catalyst (expressed in metal)/oxidizing agent molar ratio ranges between 1/10⁵ and 100/1, **in that** the reaction starts at ambient temperature and atmospheric pressure and **in that** the aqueous solution pH is kept under 12 and preferably ranges between 4 and 9.

4. Method according to anyone of claims 1 to 3, **characterized in that** the metal phtalocyanine is an iron phtalocyanine, possibly substituted by alkyl groups comprised of 1 to 4 carbon atoms and/or sulfonic groups in an aqueous solution,

5. Method according to anyone of claim 1 to 3, **characterized in that** the metal phtalocyanine is an iron phtalocyanine, possibly substituted by alkyl groups comprised of 1 to 4 carbon atoms and/or sulfonic groups, supported over a solid phase chosen from the group of refractory metal oxides such as alumina, zirconia, silica, tungstate, clays and organic resins such as cationic resins functionalized by ammonium groups.

6. Method according to anyone of 1 to 3, **characterized in that** the titanium catalyst is chosen from the group comprised of titanium zeolite without extra-network titanium, with a pore diameter greater than or equal to 0,65 nm, preferably titanium beta zeolite, and titanium mesoporous composites.

7. Method according to claim 6, **characterized in that** the metal catalyst (expressed in metal)/oxidizing agent molar ratio varies from 1 /10 to 1/40.

8. Method according to anyone of claims 1 to 7, **characterized in that** the oxidizing agent is a compound with a general formula of R₁OOR₂, where R₁ and R₂ are chosen identical or different from the group comprised of the hydrogen atom, the alkyl groups, linear or branched, comprised of 1 to 30 carbon atoms.

9. Method according to claim 8, **characterized in that** the oxidizing agent is chosen from the group comprised of hydrogen peroxide, hydroperoxide terbutyl and peroxide terbutyl.

10. Method according to claims 8 or 9, **characterized in that** the oxidizing agent is hydrogen peroxide.

11. Method according to anyone of claims 1 to 7, in that the oxidizing agent is a peracid with a formula of R₃COOOH, where R₃ is hydrogen or a linear or branched alkyl group comprised of 1 to 30 carbon atoms.

12. Method according to claim 11, **characterized in that** the oxidizing agent is chosen from the group comprised of peracetic acid, performic acid and perbenzoic acid.

13. Method according to anyone of claims 1 to 12, **characterized in that** the aqueous solution/hydrocarbons mass ratio ranges between 10/1 and 1/10, and preferably between 2/1 and 1/5.

14. Application of the method according to anyone of claims 1 to 13, to the desulfurizing of hydrotreated gas oils, kerosenes and gasolines, in particular gasolines derived from catalytic cracking.

## Patentansprüche

1. Verfahren zum selektiven Entschwefeln von Thiophenverbindungen, die in den von der Destillation von raffineriertem oder nicht raffineriertem Rohöl stammenden Kohlenwassertoffen enthalten sind, bestehend aus Oxidier der Thiophen-Schwefelatome zu Sulfon in Gegenwart eines Oxidationsmittels und aus Abtrennen der Sulfonverbindungen von den Kohlenwasserstoffen, **dadurch gekennzeichnet, dass** man die Thiophenverbindungen in einem eine Kohlenwasserstoffphase und eine wässrige Phase umfassenden zweiphasigen, bewegten Medium in der Gegenwart von mindestens einem in mindestens einer des zwei Phasen löslichen oxidationsmittel, das aus der aus organischen Peroxiden, Wasserstoffperoxid, organischen Hydro-peroxiden, Persäuren und den Alkali- und Erdalkalipersulfaten bestehenden Gruppe ausgewählt ist, und mindestens einem metallkatalysator, der aus den Metallphthalocyaninen, die gegebenenfalls mit aus 1 bis 4 Kohlenstoffatomen bestehenden Alkylgruppen und/oder Sulfonsäuregruppen substituiert sind, und Katalysatoren, die einen Träger aus der aus Siliziumoxiden, Aluminiumoxiden, Zirkonoxiden, amorphen oder kristallinen Altiminiumsilikaten, Aluminiumphosphaten und mesoporösen Feststoffen - für sich alleine oder gemischt - bestehenden Gruppe umfassen, gegebenenfalls mindestens ein Metall der aus der aus Titan, Zirkon, Vanadium, Chrom, Molybdän, Wolfram, Eisen, Magnesium und Wolfram bestehenden Gruppe umfassend, wobei diese Metalle in das Trägernetzwerk eingebracht werden können oder in Form eines Komplexes oder in nicht-komplexierter Form imprägniert werden, wobei die nicht-oxidierten Titankatalysatoren bevorzugt sind, ausgewählt wird, wobei sich die Abtrennung gleichzeitig zur Oxidation durch Extraktion ereignet, das heißt durch den Übergang der sulfonierten Thiophenverbindungen von der Kohlenwasserstoffphase in die wässrige Phase.

2. Verfahren gemäß Anspruch 1, **dadurch gekennzeichnet, dass** man ein aus der aus 1 bis 4 Kohlenstoffatome umfassenden Alkoholen, Acetonitril, Dimethylformamid, Nitromethan, Nitrobenzol bestehenden Gruppe ausgewähltes Extraktionslösungsmittel zu dem zweiphasigen Reaktiorismedium in einem Wasser/Lösungsmittel-Verhältnis, das zwischen 1/99 und 99/1 und bevorzugt zwischen 25/75 und 75/25 liegt, zugibt.

3. Verfahren gemäß einem der Ansprüche 1 und 2, **dadurch gekennzeichnet, dass** das Molverhältnis von Metallkatalysator (ausgedrückt in Metall)/Oxidationsmittel zwischen 1/10⁵ und 100/1 liegt, so dass die Reaktion bei Raumtemperatur unter Atmosphärendruck beginnt und dass der pH der wässrigen Lösung unter 12 und bevorzugt zwischen 4 und 9 gehalten wird.

4. Verfahren gemäß einem der Ansprüche 1 bis 3, **dadurch gekennzeichnet, dass** das Metallphthalocyanin ein Eisenphthalocyanin in einer wäßrigen Lösung ist, das eventuell, mit 1 bis 4, Kohlenstoffatome umfassenden Alkylgruppen und/oder Sulfongruppen substituiert ist.

5. Verfahren gemäß einem der Ansprüche 1 bis 3, **dadurch gekennzeichnet, dass** das Metallphthalocyanin ein Eisenphthalocyanin ist, das eventuell mit 1 bis 4 Kohlenstoffatome umfassenden Alkylgruppen, und/oder Sulfongruppen substituiert ist, das über eine aus der Gruppe aus hitzebeständigen Metalloxiden wie Aluminiumoxid, Zirkonoxid, Siliziumoxid, Wolframat, Tonen und organischen Harzen wie mit Ammoniumgruppen funktionalisierte kationische Harze ausgewählten Festphase getragen wird.

6. Verfahren gemäß einem der Ansprüche 1 bis 3, **dadurch gekennzeichnet, dass** der Titankatalysator aus der aus den Titan-Zeolithen ohne zusätzliches Netzwerktitan mit einem Porendurchmesser von größer oder gleich 0,65 nm bestehenden Gruppe ausgewählt wird, bevorzugt Titan-beta-Zeolith und titanhaltige mesoporöse Verbundstoffe.

7. Verfahren gemäß Anspruch 6, **dadurch gekennzeichnet, dass** das Molverhältnis von Metallkatalysator (ausgedrückt in Metall)/Oxidationsmittel von 1/10 von 1/10 bis 1/40 reicht.

8. Verfahren gemäß einem der Ansprüche 1 bis 7, **dadurch gekennzeichnet, dass** das Oxidationsmittel eine Verbindung mit der allgemeinen Formel R₁OOR₂ ist, in welcher R₁ und R₂ gleich oder verschieden aus der aus dem Wasserstoffatom und den linearen oder verzweigten, aus 1 bis 30 Kohlenstoffatomen bestehenden Alkylgruppen ausgewählt werden.

9. Verfahren gemäß Anspruch 8, **dadurch gekennzeichnet, dass** das Oxidationsmittel aus der aus Wasserstoffperoxid, Tertbutylhydroperoxid und Tertbutylperoxid bestehenden Gruppe ausgewählt wird.

10. Verfahren gemäß einem der Ansprüche 8 und 9, **dadurch gekennzeichnet, dass** das Oxidationsmittel Wasserstoffperoxid ist.

11. Verfahren gemäß einem der Ansprüche 1 bis 7, **dadurch gekennzeichnet, dass** das Oxidationsmittel eine Persäure mit der Formel ist, in welcher R₃ Wasserstoff oder eine 1 bis 30 Kohlenstoffatome umfassende lineare oder verzweigte Alkylgruppe ist.

12. Verfahren gemäß 11, **dadurch gekennzeichnet, dass** das Oxidationsmittel aus der aus Peressigsäure, Perameisensäure und Perbenzoesäure bestehenden Gruppe ausgewählt ist.

13. Verfahren gemäß einem der Ansprüche 1 bis 12, **dadurch gekennzeichnet, dass** das Massenverhältnis von wässriger Lösung/Kohlenwasserstoffen zwischen 10/1 und 1/10, und bevorzugt zwischen 2/1 und 1/5 liegt.

14. Verwendung des Verfahrens gemäß einem der Ansprüche 1 bis 13 zur Entschwefelung von hydrobehandelten Dieselölen, Kerosinen und Benzinen, insbesondere von vom katalytischen Cracken stammenden Benzinen.
